# EUROPEAN PATENT APPLICATION

(11) **EP 3 403 648 A1**
(43) Date of publication of application: **21.11.2018**
(21) Application number: 17192172.9
(22) Date of filing: 20.09.2017
(51) Int. Cl.: A61K 9/20, A61K 31/14, A61K 31/155

(54) **COMPOUND FOR TREATING OPEN INJURY, AND PREPARATION METHOD AND APPLICATION THEREOF**

(30) Priority: 17.05.2017 CN 201710349180
(71) Applicant: Beijing Textile Medical Technology & Development Co., Ltd., Beijing (CN)
(72) Inventor: ZHANG, JINYU, Beijing (CN); WANG, FENG, Beijing (CN)
(74) Representative: Roman, Alexis

(57) **Abstract**

The present disclosure pertains to the technical field of medicine, and particularly relates to a compound for treating open injury, and a preparation method and an application thereof. The compound for treating open injury is prepared from the following raw materials: a guanidine disinfectant and/or a quaternary ammonium disinfectant, and a water-soluble anionic cellulose and a derivative thereof. The compound for treating open injury of the present invention may reduce the time of exposing a wound to be minimum in various emergency situations, to reduce the number of bacteria and bacterial endotoxins to a maximum extent. The compound for treating open injury of the present invention is convenient to store, carry and use, is dedicated in the treatment field of open injury, and has a wide application prospect.

## Description

### Technical Field

The present disclosure pertains to the technical field of medicine, and particularly relates to a compound for treating open injury, and a preparation method and an application thereof. The compound for treating open injury may be used for treating open injury.

### Background

Contrary to closed injury, open injury, as the name implies, is injury causing internal tissue of the injured part (such as muscle and bone) to communicate with the outside; and in short, is injury causing blood to flow out or muscle or bone to be exposed, for example, abrasions, avulsions, cut wounds, puncture wounds and the like. Closed injury generally refers to injury without blood, such as pulled muscle and the like. Open injury is common in peacetime or wartime. Because of being polluted frequently, if not dressed in time or properly, wounds are easily infected, which affects healing and function recovery, and causes wounded personnel to be maimed and even threats the life of wounded personnel. Open injury and closed injury are mainly used in the wound examination report.

In general, the pathological change of open injury caused by serious external force may be divided into three areas: the first area is a direct contact area on the surface or center, in which foreign matter persistence or issue necrosis may exist. The second area is a surrounding area, in which damage of tissue of each layer may cause necrosis and cause infection easily if not excided. The third area is a peripheral tissue concussion reaction area having change in oedema, exudation, vasospasm, low cell viability, which may return to normal if no infection occurs, but symptom aggravates if infection occurs. Wound tract caused by firearm injury may also be divided into three areas from the inside to the outside, wherein the primary wound tract area is a direct injury area having change in devitalized tissue, foreign matter, blood clod and exudation. The area closely adjacent to the periphery of the wound tract is a contusion area, in which some or all tissue may be necrosed. The outermost area is a congestion area having change in blood circulatory disorder, oedema, exudation and blood stasis.

The open injury is dressed to improve the repair condition and prompt healing as soon as possible. According to injury conditions, polluted and infected wounds and war wounds are respectively dressed and cleaned.

Main factors of wound infection include: wound exposure time, number of wound position bacteria, and number of wound position bacteriotoxin. Conducting disinfection treatment on open injury as soon as possible is a key factor to prevent wound infection.

Selection standards for local disinfectants of wounds include: having extensive sterilization power, not being easily absorbed by organism when being locally used, having no anaphylaxis to the wound and organism, and having safe concentration when being locally used.

Chemical disinfectants frequently used in the world at present mainly include: (1) aldehyde: including formaldehyde, glutaraldehyde; (2) alkylated gas: ethylene oxide; (3) halogen: iodine, chlorine; (4) alcohol: ethanol; (5) phenol: lysol; (6) guanidine: chlorhexidine, polyhexamethylene guanidine; (7) quaternary ammonium salt: benzalkonium bromide, Benzalkonium chloride and domiphen bromide; and (8) peroxide: peracetic acid, wherein aldehyde, phenol, alcohol and peroxide are not suitable for open injury because of having strong irritation, alkylated gas is not suitable for open injury as well because of being gaseous at room temperature and not easy to use, and halogen is not suitable for being prepared into products for long-term use because of poor stability; and guanidine and quaternary ammonium disinfectants are suitable for being prepared into medical products because of meeting the basic requirements for wounds.

Disinfection products for open injury at present mainly include:
The first one, a product containing a medical gel with a disinfectant. This type of product cannot absorb blood, body fluid and pus exuded from an open wound because of containing a large amount of water, thus the disinfectant in the formula cannot fully come into contact with the wound, and is easily diluted by extravasate, so that the efficient sterilization concentration at the wound position cannot be kept. The formula also contains various carbohydrate, amino acids and electrolyte material, providing nutritive material for microorganism and being not beneficial to keeping the sterile environment of the wound.

For example, the patent CN105055304A discloses a rinse-free skin-care disinfection gel containing composite sterilization ingredients. The technical solution thereof is that: a humectant is immersed in deionized water two days ahead, to dissolve same for later use; a thickener is stirred and dissolved in water one day ahead for later use; an antibacterial agent is dissolved in ethanol at room temperature, a quaternary ammonium disinfectant and a biguanides disinfectant are added therein, then the completely dissolved thickener solution and the dissolved humectant solution are added therein, after uniformly stirring same, the pH value is adjusted to be 5.0-7.5 using a neutralizer, deionized water is added to the total amount, and solidification is performed after uniformly stirring same, so that the rinse-free skin-care disinfection gel of the present invention is obtained. In the application, this type of product cannot absorb blood, body fluid and pus exuded from an open wound because of containing a large amount of water, thus the disinfectant in the formula cannot fully come into contact with the wound, and is easily diluted by extravasate, so that the efficient sterilization concentration at the wound position cannot be kept.

For example, the patent 103493852A discloses a traditional Chinese medicine disinfection gel, every 100 parts by weight of disinfection solution thereof contain the following ingredients by wt.%: 60-70 parts by weight of extract of traditional Chinese medicine, 0.3-0.35 part by weight of trichloro hydroxydiphenyl ether, 2-5 parts by weight of hydroxy propyl methyl cellulose, 1-3 parts by weight of glycerine, 60-70 parts by weight of ethanol, and water as the rest; the raw medicine of the composite traditional Chinese medicine contains the following ingredients by wt.%: 20-25 parts by weight of chrysanthemum, 10-15 parts by weight of root of ballonflower, 10-15 parts by weight of climbing groundsel herb, 10-15 parts by weight of dandelion, 5-10 parts by weight of Chinese gall, 15-20 parts by weight of gentian, 10-15 parts by weight of Fructus Xanthii, 15-20 parts by weight of pine needle, 10-15 parts by weight of angelica dahurica, 7-12 parts by weight of scutellaria, 15-20 parts by weight of glabrous greenbrier rhizome, 15-20 parts by weight of bupleurum, 7-12 parts by weight of chuanxiong, 5-10 parts by weight of cynanchum paniculatum and 5-10 parts by weight of cyrtomii rhizoma. The gel product formula contains a large number of traditional Chinese medicine extracts. These extracts contain various carbohydrate, amino acid and electrolyte materials, providing nutritive materials for microorganism and being not beneficial to keeping the sterile environment of the wound.

The second one, the disinfectant is mixed with a film-forming agent. When being used at the wound position, film can be quickly formed to seal wound surface. Similarly, blood, body fluid and pus exuded from an open wound cannot be absorbed. The quick drying characteristic of the film-forming agent in the formula may seal a large amount of disinfectant inside the film-forming agent, so that the expected effect may not be produced. Only the disinfectant on the surface layer of the product may fully come into to contact with the wound, and is easily diluted by extravasate, so that the efficient sterilization concentration at the wound position cannot be kept.

The third one, the disinfectant is mixed with various traditional Chinese medicine ingredients, and hemostatic gauze is immersed in the mixture. Because the disinfectant is only physically mixed with other ingredients, the phenomenon that the concentration gradient of the disinfectant is the maximum and the release rate is too fast at the initial stage of hemostasis, and the concentration is reduced at the later stage after a large amount of disinfectant is released. Therefore, the risk of poisoning due to excessive absorption or relatively low concentration at the wound position may occur. The formula contains complicated traditional Chinese medicine ingredients including various nutritional ingredients such as carbohydrate, protein, lipid and the like, providing nutritive materials for microorganism and being not beneficial to keeping the sterile environment of the wound. For example, the patent 104524621A discloses a lotus root powder hemostatic gauze, prepared from the following raw materials by wt.%: 6-8 parts by weight of lotus root powder, 2-4 parts by weight of tapioca, 6-8 parts by weight of gelatin, 1-2 parts by weight of propylene glycol, 2-4 parts by weight of sodium alginate, 2-4 parts by weight of sodium carboxymethyl cellulose, 0.5-1 part by weight of tartaric acid, 1-2 parts by weight of perilla herb oil, 0.3-0.5 part by weight of isopropyl myristate, -800.1-0.2 part by weight of polysorbate, 0.01-0.03 part by weight of methyl parahydroxybenzoate, 1-2 parts by weight of auxiliary material, and 200-250 parts by weight of water.

### Summary

To solve the above-mentioned problem, the present invention provides a compound for treating open injury.

Another object of the present invention is to provide a method for preparing the compound for treating open injury.

Another object of the present invention is to provide an application of the compound for treating open injury.

The present invention is realized by the following technical solution:
A compound for treating open injury, prepared from the following raw materials: a guanidine disinfectant and/or a quaternary ammonium disinfectant, and a water-soluble anionic cellulose and a derivative thereof.

In the compound for treating open injury, the guanidine disinfectant is one or more of a chlorhexidine or a pharmaceutically acceptable salt thereof, and a polyhexamethylene guanidine or a pharmaceutically acceptable salt thereof; the quaternary ammonium disinfectant is one or more of benzalkonium chloride, benzalkonium bromide and domiphen bromide; and the derivative of water-soluble anionic cellulose is one or two of partially cross-linked products of sodium carboxymethyl cellulose or carboxymethyl cellulose.

Preferably, in the compound for treating open injury, the pharmaceutically acceptable salt of the chlorhexidine is chlorhexidine hydrochloride, chlorhexidine acetate or chlorhexidine digluconate; and the pharmaceutically acceptable salt of polyhexamethylene guanidine is polyhexamethylene guanidine hydrochloride, polyhexamethylene guanidine sulfate or polyhexamethylene guanidine stearate.

The chlorhexidine or the pharmaceutically acceptable salt thereof/sodium carboxymethyl cellulose compound contains 10mg-80mg of chlorhexidine in each g of compound by chlorhexidine.

The polyhexamethylene guanidine or the pharmaceutically acceptable salt thereof/sodium carboxymethyl cellulose compound contains 7mg-30mg of polyhexamethylene guanidine in each g of compound by polyhexamethylene guanidine.

The benzalkonium chloride sodium carboxymethyl cellulose compound contains 4mg-20mg of benzalkonium chloride in each g of compound.

The benzalkonium bromide sodium carboxymethyl cellulose compound contains 4mg-20mg of benzalkonium bromide in each g of compound.

The domiphen bromide sodium carboxymethyl cellulose compound contains 10mg-50mg of domiphen bromide in each g of compound.

A method for preparing the compound for treating open injury, comprising the following steps: adding a guanidine disinfectant and/or a quaternary ammonium disinfectant into an ethanol solution with the concentration of 65%-90% at room temperature, to dissolve same completely, adding a water-soluble anionic cellulose or a derivative thereof therein, stirring or ultrasonically oscillating to make same react completely, taking out a wet compound, and drying the wet compound, so that the compound is obtained.

An application of the compound for treating open injury in preparation of medical products for treating open injury, wherein the medical products may be medicine or medical apparatus and instruments.

The present invention has the following beneficial effects:
In the compound for treating open injury of the present invention, disinfectants are screened, and guanidine and quaternary ammonium disinfectants having extensive sterilization power, not being easily absorbed by organism when being locally used, having no anaphylaxis to wound and organism, and having safe concentration when being locally used are selected finally.

In the compound for treating open injury of the present invention, the guanidine and quaternary ammonium disinfectants and the water-soluble anionic cellulose derivative are prepared into composite disinfection hemostasis material under a certain technological condition. The ionic states of the guanidine and quaternary ammonium disinfectants both represent electropositivity. The ionic states of the water-soluble anionic cellulose and the derivative thereof both represent electronegativity. In a system capable of providing an appropriate ionic environment solvent, the guanidine and quaternary ammonium disinfectants react with the water-soluble anionic cellulose derivative to generate compounds bonded through weak ionic bonds. The product is cleaned, desalted and purified, is vacuum dried to remove the solvent to obtain a compound with stable physicochemical properties, and then is sterilized, to obtain a final product.

The compound for treating open injury of the present invention has good particular properties in treatment of open injury. The compound for treating open injury of the present invention is a sterile product obtained through vacuum drying and sterilization, has the characteristic of overlong quality assurance, and may not produce secondary pollution to the wound. More importantly, the water-soluble anionic cellulose derivative group in the compound after vacuum drying may quickly absorb blood, body fluid, pus and the like at the wound position. Meanwhile, swelling and dissolution occur. In the ionic environment formed by various extravasate from the wound, the weak ionic bonds between the compounds are opened, so that the disinfectant with electropositivity is released. This process has a positive correlation with the amount of various extravasate generated from the wound. During treatment, the compound for treating open injury of the present invention may implement the function of controlling and releasing disinfectants. In the process of treatment, the compound for treating open injury of the present invention may not substantially reduce the effective concentration of the disinfectant at the wound position due to the increase of the amount of blood and other liquid; and may not release a large amount of disinfectant at the initial stage of treatment to exceed the safe concentration of disinfectant permitted to be used at the wound position, and may not cause adverse effects such as anaphylaxis to local wound and the whole body, poisoning and the like.

All raw materials including guanidine and quaternary ammonium disinfectants and the water-soluble anionic cellulose derivative used for preparing the compound for treating open injury of the present invention may conform to the national standards or the standards of Chinese Pharmacopoeia, and reliable quality control standards and analysis methods may guarantee that the raw materials have qualified quality, thereby finally reducing the risk of the compound for treating open injury of the present invention in implementation. In the case where the wound healing is good, for the compound for treating open injury of the present invention, the rest part may be dissolved and removed using purified water, normal saline or the like. No adverse effect may be produced on the healed wound, and no secondary damage may be caused to the wound due to removal of the treatment material. The compound for treating open injury of the present invention may be prepared into sterile products, and the ingredients used in the formula are simple, stable in physicochemical properties, and low in moisture content, thereby being beneficial to long-term storage of the product. The compound for treating open injury of the present invention is small in volume, and light in unit weight (1 g of the product may completely cover and treat the conventional open injury). The compound for treating open injury of the present invention may reduce the time of exposing a wound to be minimum, to reduce the number of bacteria and bacterial endotoxin to a maximum extent. The compound for treating open injury of the present invention is convenient to store, carry and use, is dedicated in the treatment field of open injury, has function and applicability superior to those of similar products, and has a wide application prospect.

### Detailed Description

The present invention will be further explained in combination with specific embodiments, so that those skilled in the art may know the present invention more, but do not limit the present invention thereby.

### Embodiment 1

A proper amount of purified water is added into 95% medical ethanol, to prepare an ethanol solution with the concentration of 65%-90%. Chlorhexidine is added at room temperature, the mixture is stirred until chlorhexidine is completely dissolved, and a chlorhexidine ethanol solution with the concentration of 0.1%-0.5% is prepared. 300L of the solution is taken and then added into a reaction kettle. 8-15 kg of sodium carboxymethyl cellulose is weighed, and is slowly added into the reaction kettle by stirring or ultrasonic oscillation at room temperature, to guarantee that the sodium carboxymethyl cellulose is completely added within 30-60 minutes. Due to heterogeneous phase system in the reaction, complete stirring or ultrasonic oscillation is required, to guarantee that reaction is completely conducted. This reaction is an exothermic reaction, so that heating is not required in the reaction process. After continuous reaction for 2-3 hours, an ethanol solution with the concentration of 65%-90% is added in three times (100 L each time), to conduct cleaning. Then, a wet compound is taken out from the reaction kettle. The wet compound is placed in a vacuum drying oven for vacuum drying at the temperature of not more than 50°C. The end point moisture content is controlled to be within 5%. The product is sterilized after being packed, and it is measured that the chlorhexidine content in the finished product is 10mg-80mg/g.

### Embodiment 2

A proper amount of purified water is added into 95% medical ethanol, to prepare an ethanol solution with the concentration of 65%-90%. 20% polyhexamethylene guanidine is added at room temperature, the mixture is stirred until polyhexamethylene guanidine is completely dissolved, and a polyhexamethylene guanidine ethanol solution with the concentration of 0.1%-0.5% is prepared. 300 L of the solution is taken and then added into a reaction kettle. 10-16 kg of sodium carboxymethyl cellulose is weighed, and is slowly added into the reaction kettle by stirring or ultrasonic oscillation at room temperature, to guarantee that the sodium carboxymethyl cellulose is completely added within 30-60 minutes. Due to heterogeneous system in the reaction, complete stirring or ultrasonic oscillation is required, to guarantee that reaction is completely conducted. This reaction is an exothermic reaction, so that heating is not required in the reaction process. After continuous reaction for 2-3 hours, an ethanol solution with the concentration of 65%-90% is added in three times (100 L each time), to conduct cleaning. Then, a wet compound is taken out from the reaction kettle. The wet compound is placed in a vacuum drying oven for vacuum drying at the temperature of not more than 50°C. The end point moisture content is controlled to be within 5%. The product is sterilized after being packed, and it is measure that the polyhexamethylene guanidine content in the finished product is 7mg-30mg/g.

### Embodiment 3

A proper amount of purified water is added into 95% medical ethanol, to prepare an ethanol solution with the concentration of 65%-90%. Benzalkonium bromide is added at room temperature, the mixture is stirred until the benzalkonium bromide is completely dissolved, and a benzalkonium bromide ethanol solution with the concentration of 0.1%-0.5% is prepared. 300L of the solution is taken and then added into a reaction kettle. 12-20 kg of sodium carboxymethyl cellulose is weighed, and is slowly added into the reaction kettle by stirring or ultrasonic oscillation at room temperature, to guarantee that the sodium carboxymethyl cellulose is completely added within 30-60 minutes. Due to heterogeneous system in the reaction, complete stirring or ultrasonic oscillation is required, to guarantee that reaction is completely conducted. This reaction is an exothermic reaction, so that heating is not required in the reaction process. After continuous reaction for 1-2 hours, an ethanol solution with the concentration of 65%-90% is added in three times (100 L each time), to conduct cleaning. Then, a wet compound is taken out from the reaction kettle. The wet compound is placed in a vacuum drying oven for vacuum drying at the temperature of not more than 50°C. The end point moisture content is controlled to be within 5%. The product is sterilized after being packed, and it is measured that the benzalkonium bromide content in the finished product is 4mg-20mg/g.

### Embodiment 4

A proper amount of purified water is added into 95% medical ethanol, to prepare an ethanol solution with the concentration of 65%-90%. Benzalkonium chloride is added at room temperature, the mixture is stirred until the benzalkonium chloride is completely dissolved, and a benzalkonium chloride ethanol solution with the concentration of 0.1%-0.5% is prepared. 300 L of the solution is taken and then added into a reaction kettle. 12-20 kg of sodium carboxymethyl cellulose is weighed, and is slowly added into the reaction kettle by stirring or ultrasonic oscillation at room temperature, to guarantee that the sodium carboxymethyl cellulose is completely added within 30-60 minutes. Due to heterogeneous system in the reaction, complete stirring or ultrasonic oscillation is required, to guarantee that reaction is completely conducted. This reaction is an exothermic reaction, so that heating is not required in the reaction process. After continuous reaction for 1-2 hours, an ethanol solution with the concentration of 65%-90% is added in three times (100 L each time), to conduct cleaning. Then, a wet compound is taken out from the reaction kettle. The wet compound is placed in a vacuum drying oven for vacuum drying at the temperature of not more than 50°C. The end point moisture content is controlled to be within 5%. The product is sterilized after being packed, and is measured that the benzalkonium chloride content in the finished product is 4mg-20mg/g.

### Embodiment 5

A proper amount of purified water is added into 95% medical ethanol, to prepare an ethanol solution with the concentration of 65%-90%. Domiphen bromide is added at room temperature, the mixture is stirred until the domiphen bromide is completely dissolved, and a domiphen bromide ethanol solution with the concentration of 0.1%-0.5% is prepred. 300 L of the solution is taken and then added into a reaction kettle. 8-15 kg of sodium carboxymethyl cellulose is weighed, and is slowly added into the reaction kettle by stirring or ultrasonic oscillation at room temperature, to guarantee that the sodium carboxymethyl cellulose is completely added within 30-60 minutes. Due to heterogeneous system in the reaction, complete stirring or ultrasonic oscillation is required, to guarantee that reaction is completely conducted. This reaction is an exothermic reaction, so that heating is not required in the reaction process. After continuous reaction for 1-2 hours, an ethanol solution with the concentration of 65%-90% is added in three times (100 L each time), to conduct cleaning. Then, a wet compound is taken out from the reaction kettle. The wet compound is placed in a vacuum drying oven for vacuum drying at the temperature of not more than 50°C. The end point moisture content is controlled to be within 5%. The product is sterilized after being packed, and it is measured that the domiphen bromide content in the finished product is 10mg-50mg/g.

Bacteriostasis experiment is conducted on the compound for treating open injury of the present invention. The details are as follows:
1. Experiment material: 5 g of compound for treating open injury as a sample for test, and 5g of non-composited sodium carboxymethyl cellulose as a control sample.
2. Experiment strains: staphylococcus aureus, pseudomonas aeruginosa, escherichia coli, candida albicans and aspergillus niger.
   Culture medium: trypticase soy agar medium, sabouraud dextrose agar medium.
3. Experiment method
   Bacterium solution preparation: the staphylococcus aureus, pseudomonas aeruginosa, escherichia coli and candida albicans are prepared into about 1 ml of bacterium suspension containing 10⁸ cfu using 0.9% sterile sodium chloride solution, the aspergillus niger is prepared into spore suspension containing 10⁸ cfu using 0.9% sterile sodium chloride solution containing 0.05% polysorb.

10 sterile test tubes of 200 ml are taken, each tube is added with 1 g of sample for test and control sample, and 100 ml of 0.9% sodium chloride solution is added therein to dissolve the sample for test. The above bacterium solution is vaccinated in each tube respectively, the amount of the vaccinated bacterium solution in each tube is 10⁶ cfu, the solution is fully mixed, and the tube is dark stored at 20-25°C. According to prescribed time, the number of survival bacteria is measured, and reduced log values are calculated according to the number of access bacteria.

**Experiment Results:**

| Sample for test group | Reduced log values | | | | Determining |
|---|---|---|---|---|---|
| Experiment bacteria | 2d | 7d | 14d | 28d | |
| Staphylococcus aureus | 6 | 6 | NR | NR | Conformity |
| Pseudomonas aeruginosa | 6 | 6 | NR | NR | Conformity |
| Escherichia coli | 5.6 | 6 | NR | NR | Conformity |
| Candida albicans | 5.1 | 6 | NR | NR | Conformity |
| Aspergillus Niger | 6 | 6 | NR | NR | Conformity |

| Control Group | Reduced log value | | | | Determining |
|---|---|---|---|---|---|
| Experiment bacteria | 2d | 7d | 14d | 28d | |
| Staphylococcus aureus | NI | NI | NI | NI | Inconformity |
| Pseudomonas aeruginosa | NI | NI | NI | NI | Inconformity |
| Escherichia coli | NI | NI | NI | NI | Inconformity |
| Candida albicans | NI | NI | NI | NI | Inconformity |
| Aspergillus Niger | NI | NI | NI | NI | Inconformity |

Experiment conclusion: the compound for treating open injury has excellent disinfection and sterilization effects; and represent killing capacity to gram-positive bacteria, gram-negative bacteria, bacterial spores, fungus and spores, and can keep the sterile effect after disinfection for a long time; the non-composited sodium carboxymethyl cellulose has no sterilization effect, but has a certain inhibiting effect on the growth of various bacteria. This experiment is conducted after the sample for test is diluted by 100 times, so that in the practical application process, after the liquid such as blood is diluted, this patent may still keep the original design function.

## Claims

1. A compound for treating open injury, prepared from the following raw materials: a guanidine disinfectant and/or a quaternary ammonium disinfectant, and a water-soluble anionic cellulose and a derivative thereof.

2. The compound for treating open injury of claim 1, **characterized in that** the guanidine disinfectant is one or more of chlorhexidine or a pharmaceutically acceptable salt thereof, and polyhexamethylene guanidine or a pharmaceutically acceptable salt thereof; the quaternary ammonium disinfectant is one or more of benzalkonium chloride, benzalkonium bromide and domiphen bromide; and the derivative of the water-soluble anionic cellulose is one or two of partially cross-linked products of sodium carboxymethyl cellulose or carboxymethyl cellulose.

3. The compound for treating open injury of claim 2, **characterized in that** the pharmaceutically acceptable salt of the chlorhexidine is chlorhexidine hydrochloride, chlorhexidine acetate or chlorhexidine digluconate; the pharmaceutically acceptable salt of the polyhexamethylene guanidine is polyhexamethylene guanidine hydrochloride, polyhexamethylene guanidine sulfate or polyhexamethylene guanidine stearate.

4. The compound for treating open injury of claim 2, **characterized in that** the chlorhexidine or the pharmaceutically acceptable salt thereof/sodium carboxymethyl cellulose compound contains 10mg-80mg of chlorhexidine in each g of compound by chlorhexidine.

5. The compound for treating open injury of claim 2, **characterized in that** the polyhexamethylene guanidine or the pharmaceutically acceptable salt thereof/sodium carboxymethyl cellulose compound contains 7mg-30mg of polyhexamethylene guanidine in each g of compound by polyhexamethylene guanidine.

6. The compound for treating open injury of claim 2, **characterized in that** the benzalkonium chloride sodium carboxymethyl cellulose compound contains 4mg-20mg of benzalkonium chloride in each g of compound.

7. The compound for treating open injury according to claim 2, **characterized in that** the benzalkonium bromide sodium carboxymethyl cellulose compound contains 4mg-20mg of benzalkonium bromide in each g of compound.

8. The compound for treating open injury of claim 2, **characterized in that** the domiphen bromide sodium carboxymethyl cellulose compound contains 10mg-50mg of domiphen bromide in each g of compound.

9. A method for preparaing the compound for treating open injury of claim 1, comprising the following steps: adding a guanidine disinfectant and/or a quaternary ammonium disinfectant into an ethanol solution with the concentration of 65%-90% at room temperature, dissolving same completely, adding a water-soluble anionic cellulose or a derivative thereof therein, stirring or ultrasonically oscillating to make same react completely, taking out a wet compound, drying the wet compound, and obtaining the compound.

10. An application of the compound for treating open injury of claim 1 in preparation of medical products for treating open injury.
